# EUROPEAN PATENT SPECIFICATION

(11) **EP 2 901 929 B1**
(45) Date of publication and mention of the grant of the patent: **03.01.2018**
(21) Application number: 13842590.5
(22) Date of filing: 26.07.2013
(51) Int. Cl.: A61B 5/15, A61B 5/00, A61B 5/157

(54) **POSITIONING TAPE**
POSITIONIERUNGSBAND
RUBAN DE POSITIONNEMENT

(30) Priority: 27.09.2012 JP 2012214169
(43) Date of publication of application: 05.08.2015
(73) Proprietor: Sysmex Corporation, Kobe-shi, Hyogo 651-0073 (JP)
(72) Inventor: OKADA, Seiki, Kobe-shi Hyogo 651-0073 (JP); OHNISHI, Yasuhito, Kobe-shi Hyogo 651-0073 (JP); ASAKURA, Yoshihiro, Kobe-shi Hyogo 651-0073 (JP)
(74) Representative: Hoffmann Eitle
(86) International application number: PCT/JP2013/070311
(87) International publication number: WO 2014/050290

(56) References cited:
- EP-A2- 2 298 177
- WO-A1-2010/098339
- JP-A- 2001 245 875
- JP-A- 2002 186 600
- JP-A- 2010 072 496
- JP-A- 2010 213 740
- JP-A- 2011 064 542

## Description

### TECHNICAL FIELD

The present invention relates to a positioning tape.

### BACKGROUND ART

A conventionally known method for acquiring biological information such as a blood sugar level includes forming a micropore in a skin of a living body and sampling tissue fluid through the micropore (see Patent Literature 1, for example). A tissue fluid collecting sheet including a collector such as gel configured to collect tissue fluid is used to sample the tissue fluid through the micropore. The micropore provided in the skin and the collector on the tissue fluid collecting sheet need to be accurately matched in position for sampling of an appropriate amount of the tissue fluid.

According to the method described in Patent Literature 1, a marker sheet is affixed at a position where micropore is to be formed as a marker for the tissue fluid collecting sheet to be affixed. More specifically, in order to form the micropore at a predetermined accurate position in the marker sheet affixed to the skin, the method described in Patent Literature 1 includes positionally matching by visual observation a cutout provided at a brim, i.e. a contact portion, of a puncture tool for formation of a micropore and a cutout provided in a marker retaining sheet for retaining the marker sheet, and then forming the micropore.

Further, Patent Literature 2 is concerned with an interstitial fluid collection sheet.

### CITATION LIST

### PATENT LITERATURE

Patent Literature 1: Japanese Unexamined Patent Publication No. 2011-64542
Patent Literature 2: EP 2 298 177 A2.

### SUMMARY OF INVENTION

### TECHNICAL PROBLEMS

Positionally matching by visual observation, however, may make it difficult to accurately find the position of the cutouts that will be behind the puncture tool depending on an affixed position of the marker retaining sheet or a direction thereof. This may lead to formation of the micropore at a wrong position and acquisition of an inaccurate test result.

The present invention has been achieved in view of these circumstances, and an objective thereof is to provide a positioning tape that enables accurate formation of a micropore at a predetermined position.

### SOLUTIONS TO PROBLEMS

A positioning tape according to the present invention is configured to indicate a region provided with a micropore when a skin is made in contact with a micropore forming device and is provided with the micropore by a microneedle of the micropore forming device, wherein the micropore forming device is contactable with the positioning tape, the positioning tape includes:
a supporter having a positioning portion including a step having a shape at least partially corresponding to a shape of a contact portion of the micropore forming device; and
a marker portion having an opening corresponding to the region provided with the micropore;
characterised in that: when the contact portion of the micropore forming device is made in contact with the positioning tape for micropore formation, the step at the positioning portion is configured to regulate shift of the micropore forming device in a skin surface direction.

When the contact portion of the micropore forming device configured to form a micropore is made in contact with the positioning tape according to the present invention, the step at the positioning portion regulates shift of the micropore forming device in the skin surface direction. The micropore forming device can be thus positioned accurately on the skin. The micropore can be accordingly formed accurately in a predetermined region in the opening in the marker portion. This improves accuracy of a test result of tissue fluid to be sampled.

The marker portion can be provided as a marker sheet separable from the supporter.

The marker sheet can have a circular shape.

The positioning portion can be provided as a sheet that is separate from the supporter and has a predetermined thickness.

The predetermined thickness is possibly from 0.1 to 10.0 mm.

The positioning portion preferably includes a plurality of sheets.

The step at the positioning portion can have a circular shape or have at least partially circular arc shape.

The positioning portion is preferably provided with a notch. The notch can be provided along a short side of the positioning tape. The positioning portion preferably includes a plurality of notches.

The notch is preferably provided in a vicinity of the marker sheet.

Preferably, the positioning tape further includes a separable sheet provided on another surface of the supporter to cover the marker portion, and the separable sheet is larger than the supporter at one of ends in a long side direction.

The supporter can have an opening corresponding to the region provided with the micropore.

The supporter can be provided with a thin film portion in the region provided with the micropore.

The thin film portion is preferably transparent.

### ADVANTAGEOUS EFFECT OF INVENTION

The positioning tape according to the present invention enables accurate formation of a micropore at a predetermined position.

### BRIEF DESCRIPTION OF DRAWINGS

[FIG. 1] FIG. 1 is an explanatory perspective view of a puncture tool used with a positioning tape according to the present invention.
[FIG. 2] FIG. 2 is a perspective view of a microneedle chip mounted to the puncture tool shown in FIG. 1.
[FIG. 3] FIG. 3 is an explanatory sectional view of a skin provided with micropores by the puncture tool.
[FIG. 4] FIG. 4 is an explanatory plan view of a positioning tape according to an embodiment of the present invention.
[FIG. 5] FIG. 5 is an exploded perspective view of the positioning tape shown in FIG. 4.
[FIG. 6] FIG. 6 is an explanatory sectional view of the positioning tape shown in FIG. 4.
[FIG. 7] FIG. 7 is a plan view of a positioning portion in the positioning tape shown in FIG. 4.
[FIGS. 8] FIG. 8(a) is a plan view of a first double-sided tape in the positioning tape shown in FIG. 4, and FIG. 8(b) is a sectional view thereof.
[FIGS. 9] FIG. 9(a) is a plan view of a single-sided tape in the positioning tape shown in FIG. 4, and FIG. 9(b) is a sectional view thereof.
[FIGS. 10] FIG. 10(a) is a plan view of spacers in the positioning tape shown in FIG. 4, and FIG. 10(b) is a sectional view thereof.
[FIGS. 11] FIG. 11(a) is a plan view of a second double-sided tape in the positioning tape shown in FIG. 4, and FIG. 11(b) is a sectional view thereof.
[FIGS. 12] FIG. 12(a) is a plan view of a marker sheet in the positioning tape shown in FIG. 4, and FIG. 12(b) is a sectional view thereof.
[FIGS. 13] FIG. 13(a) is a plan view of a third double-sided tape in the positioning tape shown in FIG. 4, and FIG. 13(b) is a sectional view thereof.
[FIG. 14] FIG. 14 is a plan view of a separable sheet in the positioning tape shown in FIG. 4.
[FIGS. 15] FIGS. 15(a) to 15(e) are views showing the steps in a tissue fluid collecting method.
[FIG. 16] FIG. 16 is an explanatory view of a use state of the positioning tape.
[FIG. 17] FIG. 17 is an explanatory perspective view of a tissue fluid collecting sheet.
[FIG. 18] FIG. 18 is a sectional view taken along the line A-A of the tissue fluid collecting sheet shown in FIG. 17.
[FIG. 19] FIG. 19 is an explanatory perspective view of outer appearance of a biological component analyzer.

### DESCRIPTION OF EMBODIMENTS

A positioning tape according to an embodiment of the present invention will now be described below with reference to the accompanying drawings.

The positioning tape according to the present invention is used for collecting tissue fluid through a micropore provided in a skin. A puncture tool used for forming such a micropore is described initially.

### [Puncture Tool]

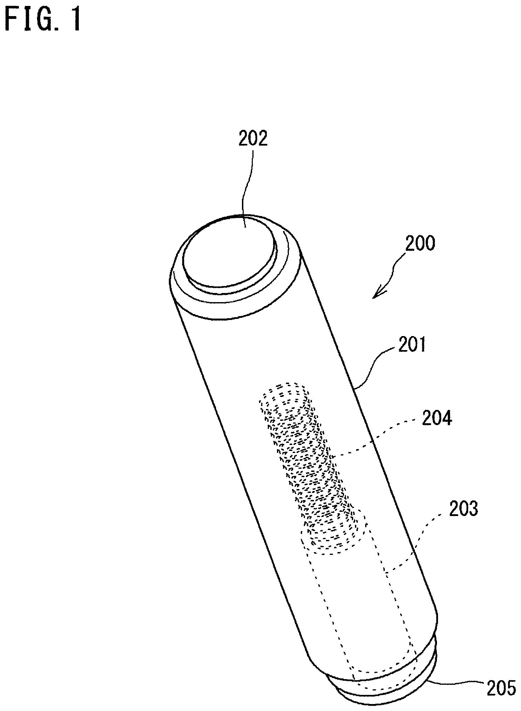

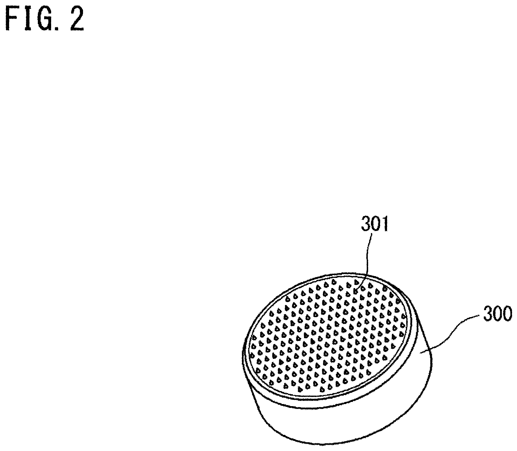

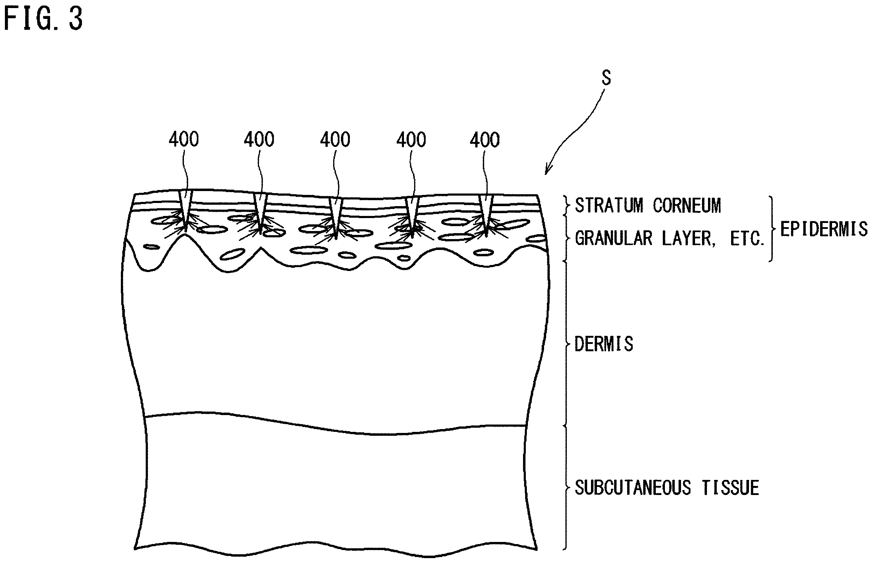

FIG. 1 is an explanatory perspective view of a puncture tool 200 serving as a micropore forming device configured to form a micropore in a skin in order to facilitate extraction of tissue fluid from the skin. FIG. 2 is a perspective view of a microneedle chip 300 mounted to the puncture tool 200 shown in FIG. 1. FIG. 3 is an explanatory sectional view of a skin S having micropores formed by the puncture tool 200.

As shown in FIGS. 1 to 3, the puncture tool 200 is mounted with the microneedle chip 300 having been sterilized. The microneedle chip 300 has microneedles 301 made in contact with an epidermis of a living body (the skin S of a test subject) to form tissue fluid extraction bores (micropores 400) in the skin S of the test subject. Each of the microneedles 301 of the microneedle chip 300 is sized such that the micropores 400 formed by the puncture tool 200 extend only in the epidermis of the skin S and do not reach a dermis.

As shown in FIG. 1, the puncture tool 200 includes a cylindrical case 201, a release button 202 provided on the upper end surface of the case 201, as well as an array chuck 203 and a spring member 204 accommodated in the case 201. The case 201 is provided, at the lower end, with a contact portion 205, and the lower end surface (in contact with the positioning tape) of the contact portion 205 has an opening (not shown) that allows the microneedle chip 300 to pass therethrough. The spring member 204 biases the array chuck 203 in a puncture direction. The microneedle chip 300 can be mounted at the lower end of the array chuck 203. The microneedle chip 300 is provided, on the lower surface, with the plurality of microneedles 301. The lower surface of the microneedle chip 300 has a circular shape. The puncture tool 200 has a fixing mechanism (not shown) configured to fix the array chuck 203 pressed upward (opposite to the puncture direction) against bias force of the spring member 204. When a user (test subject) presses downward the release button 202, the fixing mechanism releases fixing of the array chuck 203, which is thus shifted in the puncture direction by the bias force of the spring member 204. The microneedles 301 of the microneedle chip 300 projected from the opening puncture the skin.

### [Positioning Tape]

Described next is the positioning tape indicating a region to be provided with micropores in the skin S of the test subject by the above puncture tool 200.

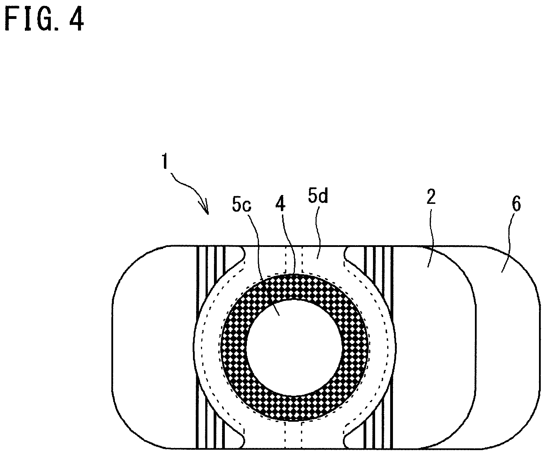

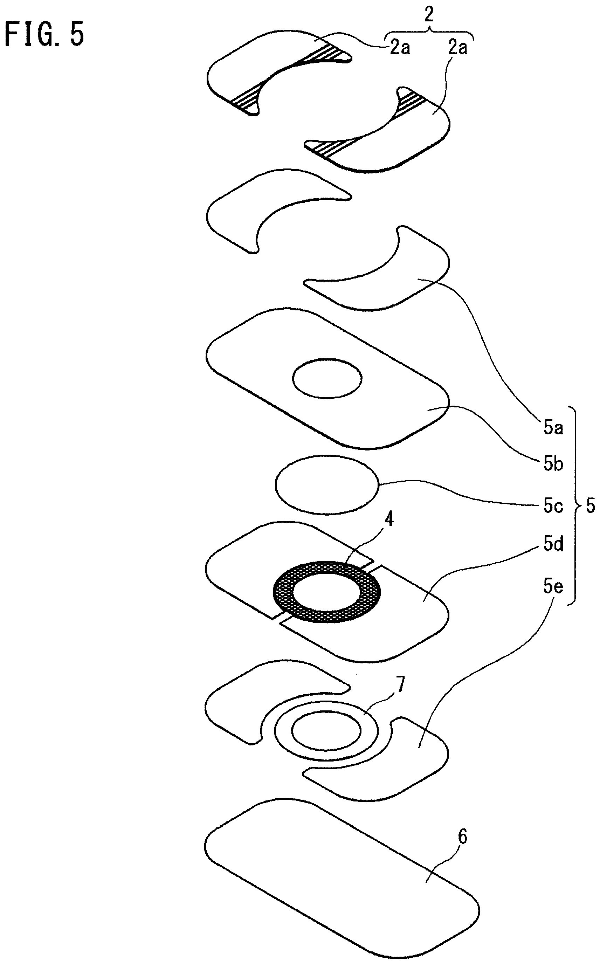

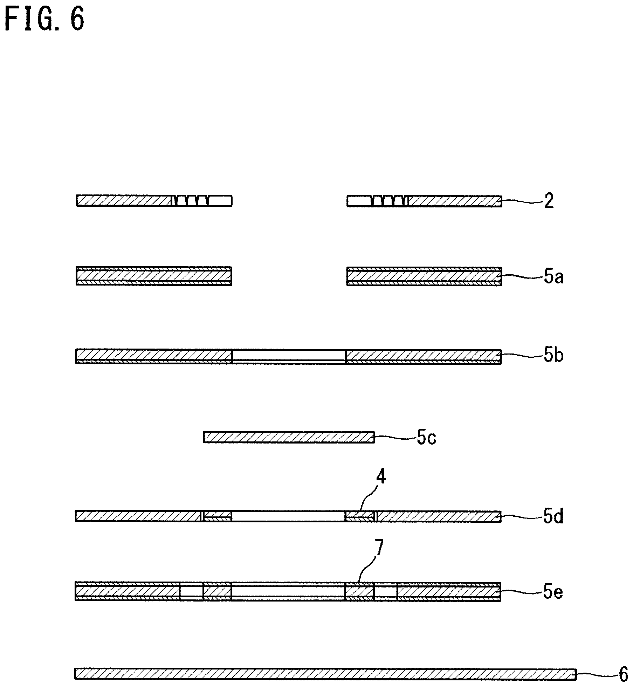

FIG. 4 is a plan view of a positioning tape 1 according to an embodiment of the present invention. FIG. 5 is an exploded perspective view of the positioning tape 1 shown in FIG. 4. FIG. 6 is an explanatory sectional view of the positioning tape 1 shown in FIG. 4. A sheet and other elements of the positioning tape 1 according to the present embodiment are quite thin members. The elements shown in FIGS. 5 and 6 are larger in thickness than the actual elements for clearer depiction. FIG. 6 shows part of sections of the elements.

The positioning tape 1 includes a positioning portion 2, a supporter 5, a marker sheet 4, and a separable sheet 6 in this order from a surface (the upper surface in FIG. 5) opposite to a surface to be affixed to the skin S of the test subject.

The respective elements are to be described below.

### <Positioning Portion>

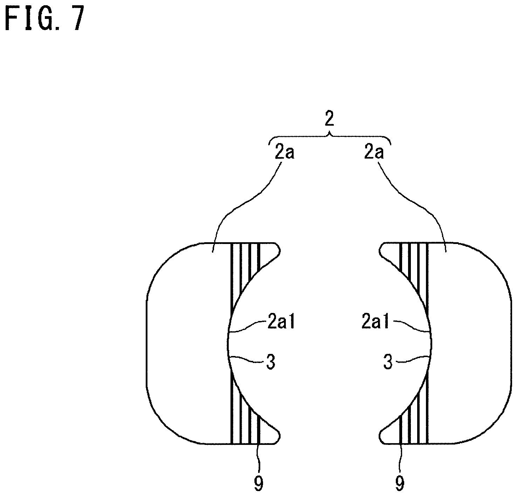

FIG. 7 is a plan view of the positioning portion 2 in the positioning tape 1 shown in FIG. 4. The positioning portion 2 is in contact with the outer peripheral surface of the contact portion 205 at the distal end of the puncture tool 200 to regulate shift of the puncture tool 200 in a skin surface direction. The positioning portion 2 according to the present embodiment includes two sheets 2a in a crescent shape. The sheets 2a are identical in material, shape, size, and the like. The sheets 2a are located on the supporter 5 such that circular arc portions 2a1 face each other.

The sheets 2a can be made of a synthetic resin mainly containing polypropylene or the like. The sheets 2a are not particularly limited in thickness in the present invention. The sheets 2a are ordinarily about 0.1 to 10.0 mm thick and are preferably 0.3 to 0.5 mm thick in view of reliable regulation of shift of the puncture tool 200 in the skin surface direction, easy removal from the skin S after use, and the like. The positioning portion 2 includes the sheets 2a having a predetermined thickness to easily form steps to be described below.

The circular arc portions 2a1 of the sheets 2a form circular arc steps 3 corresponding to the outer shape of the circular contact portion 205 of the puncture tool 200. A circular region defined by the circular arc steps 3 of the sheets 2a is set to be slightly larger (e.g. larger by about 1.0 mm) than the outer diameter of the contact portion 205 of the puncture tool 200. The distal end surface of the contact portion 205 of the puncture tool 200 can be thus easily located in the circular region defined by the circular arc steps 3. Furthermore, the contact portion 205 of the puncture tool 200 tentatively located in the circular region can be shifted in the skin surface direction by a smaller distance, and micropores can be formed reliably in a micropore formation region determined by the guide sheet 4 to be described later. The steps 3 are provided to have a circular arc shape in the present embodiment. A user is not required to consider the direction of the puncture tool 200 upon positioning the contact portion 205 of the puncture tool 200 to the steps 3, and can thus easily position the puncture tool 200.

The sheets 2a in the positioning portion 2 are provided with a plurality of notches 9 in the present embodiment. The notches 9 are provided in the vicinity of the periphery of the guide sheet 4 to be described later, so as to be along the short sides of the positioning tape 1 in a substantially rectangular shape. The positioning portion 2 needs to have a certain thickness so as to form the steps 3 regulating shift of the puncture tool 200, and has predetermined rigidity. When the positioning tape 1 is removed from the skin S with the guide sheet 4 remaining on the skin S after formation of micropores, the rigidity may makes it difficult to remove the positioning tape 1. The sheets 2a having the notches 9 according to the present embodiment are easily deformed along the notches 9 and the positioning tape 1 can be thus easily removed from the skin S. Moreover, the notches 9 provided in the vicinity of the periphery of the marker sheet 4 to be described later advantageously causes the marker sheet 4 to be easily removed from the supporter 5 and be likely to remain on the skin S.

The positioning portion 2 according to the present embodiment includes not a single sheet but the two sheets 2a. The positioning tape 1 can be thus removed more easily from the skin S.

### <Supporter>

The supporter 5 supports and holds the positioning portion 2 and the marker sheet 4 to be described later. The positioning portion 2 is affixed to one of the surfaces of the supporter 5 whereas the marker sheet 4 is affixed to the other surface.

As shown in FIGS. 5 and 6, the supporter 5 includes first double-sided tapes 5a, a single-sided tape 5b, a thin film portion 5c, spacers 5d, and second double-sided tapes 5e in this order from a surface (the upper surface in FIG. 5) opposite to a surface to be affixed to the skin S of the test subject.

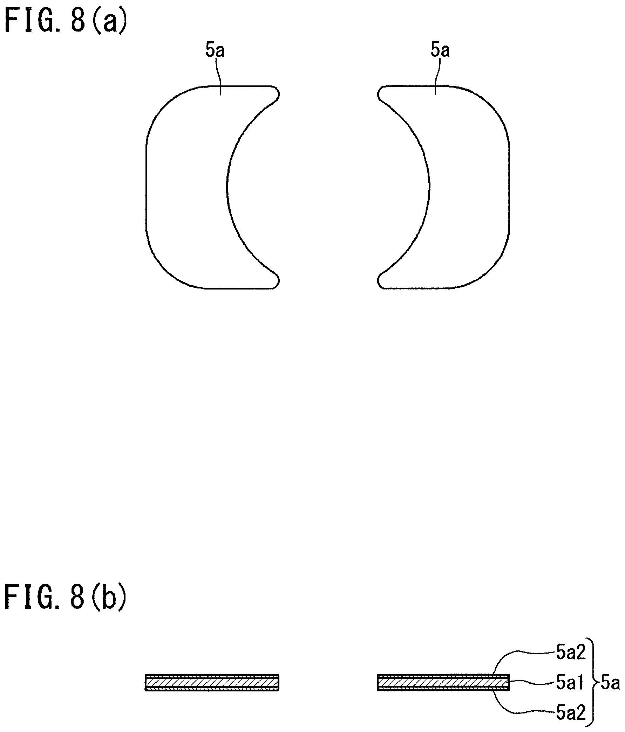

FIG. 8(a) is a plan view of the first double-sided tapes 5a in the positioning tape 1 shown in FIG. 4, and FIG. 8(b) is a sectional view thereof. Each of the first double-sided tapes 5a has a planar shape substantially same as that of the sheets 2a in the positioning portion 2, and includes a base material 5a1 that is made of a synthetic resin such as polyethylene terephthalate (PET) and is about 0.01 to 0.30 mm thick, and adhesive layers 5a2 that are provided on the both surfaces of the base material 5a1 and are made of an acrylic adhesive agent.

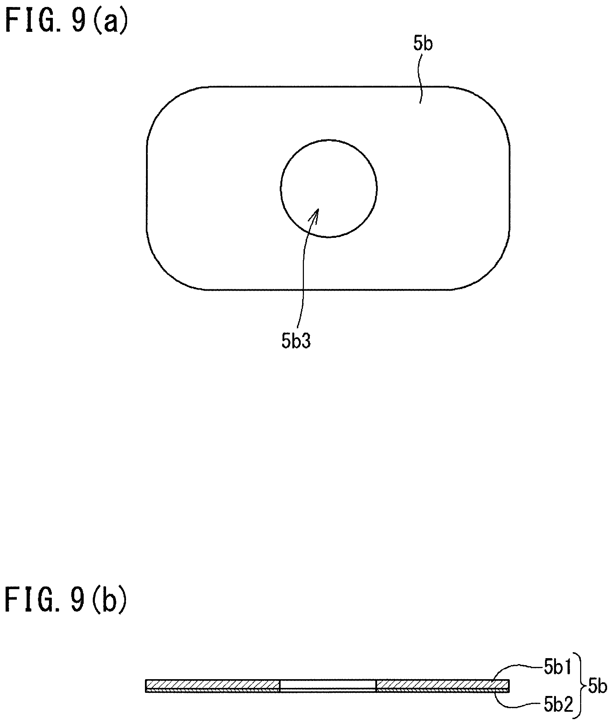

FIG. 9(a) is a plan view of the single-sided tape 5b in the positioning tape 1 shown in FIG. 4, and FIG. 9(b) is a sectional view thereof. The single-sided tape 5b includes a substantially rectangular base material 5b1 that has four chamfered corners and an adhesive layer 5b2 that is provided on a surface (opposite to the surface closer to the positioning portion 2) of the base material 5b1 and is made of an acrylic adhesive agent. The base material 5b1 can be made of a synthetic resin such as polyethylene terephthalate (PET). The base material 5b1 is not particularly limited in thickness in the present invention, but is ordinarily about 0.01 to 0.30 mm thick.

An opening 5b3 is provided at the center of the single-sided tape 5b. The opening 5b3 is sized to allow the microneedle chip 300 of the puncture tool 200 to pass therethrough (e.g. 11 to 13 mm in diameter), and has a circular shape substantially as large as the circular region determined by the marker sheet 4 to be described later.

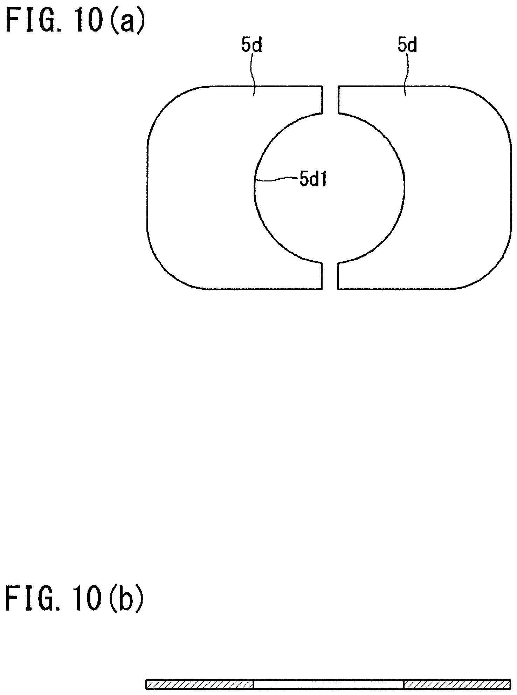

FIG. 10(a) is a plan view of the spacers 5d in the positioning tape 1 shown in FIG. 4. Each of the spacers 5d is a rectangular sheet provided, on one of the long sides, with a semicircular cutout 5d1, and can be made of a synthetic resin such as polyethylene terephthalate (PET). The spacers 5d are not particularly limited in thickness in the present invention, but are ordinarily about 0.1 to 0.5 mm thick.

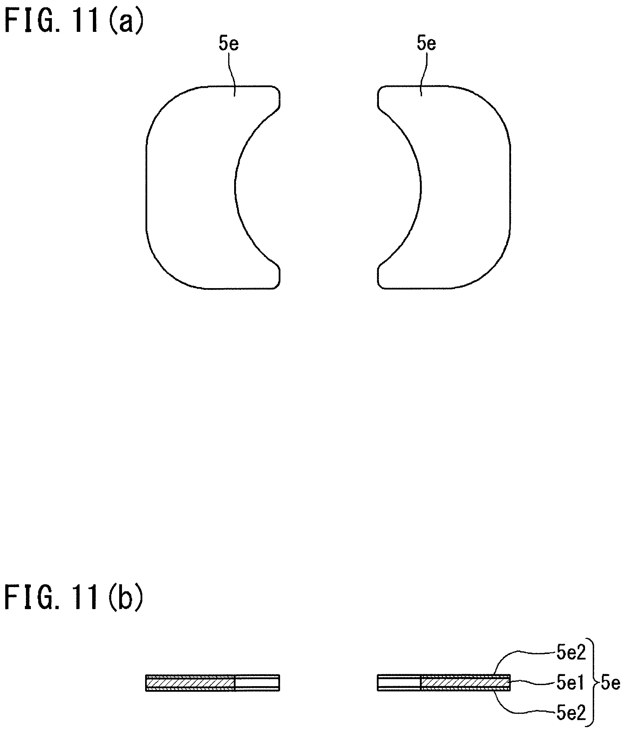

FIG. 11(a) is a plan view of the second double-sided tapes 5e in the positioning tape 1 shown in FIG. 4, and FIG. 11(b) is a sectional view thereof. Each of the second double-sided tapes 5e also has a planar shape substantially same as that of the sheets 2a in the positioning portion 2, and includes a base material 5e1 that is made of a synthetic resin such as polyethylene and is about 0.01 to 0.30 mm thick, and adhesive layers 5e2 that are provided on the both surfaces of the base material 5e1 and are made of a synthetic rubber adhesive agent.

In the present embodiment, the thin film portion 5c sized to cover the opening 5b3 in the single-sided tape 5b is provided between the single-sided tape 5b and the marker sheet 4. The thin film portion 5c is provided as a thin transparent film made of a synthetic resin such as polyethylene terephthalate (PET) or a urethane synthetic resin and is about 0.01 mm thick. The thin film portion 5c reduces impact of the microneedle chip 300 to the skin S of the test subject upon formation of micropores. A film selected as the thin film portion 5c has a thickness or strength that enables formation of a micropore in the skin S through the thin film portion 5c. The thin film portion 5c is removed from the skin S together with the positioning portion 2 and the like after micropores are formed. The transparent thin film portion 5c allows the positioning tape 1 to be affixed to the skin S of the test subject without covering an injured region for formation of micropores.

### <Marker Sheet>

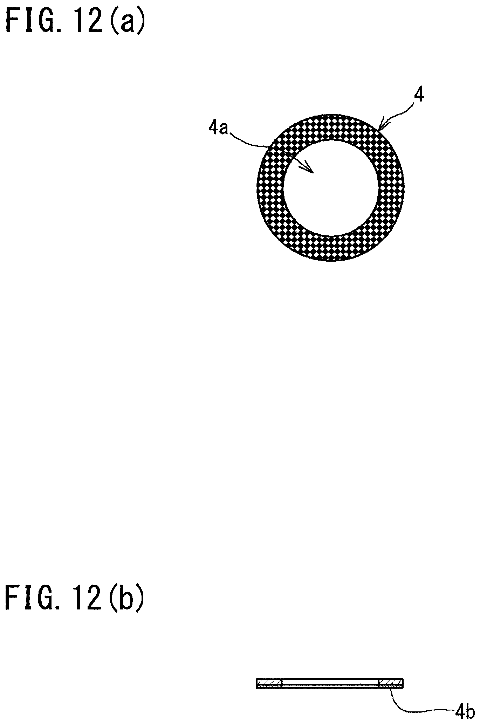

FIG. 12(a) is a plan view of the marker sheet 4 in the positioning tape 1 shown in FIG. 4, and FIG. 12(b) is a sectional view thereof. The marker sheet 4 indicates a region provided with the micropores 400 by the puncture tool 200. In the present embodiment, the positioning tape 1 including other positioning portion 2 and the like is removed from the skin S of the test subject whereas the marker sheet 4 remains affixed to the skin S of the test subject.

The marker sheet 4 has a circular shape and is provided, at the center, with an opening 4a. One (affixed to the skin S) of the surfaces of the marker sheet 4 is a printed surface 4b appropriately provided with a pattern or a color so that the region provided with micropores can be visually recognized with ease. Indication of the region provided with micropores can be achieved not by printing but by a large number of microgrooves formed in the surface of the sheet. The marker sheet 4 according to the present embodiment has the circular shape with no corners like a rectangular sheet, and will be quite unlikely to be removed from the skin S unintentionally.

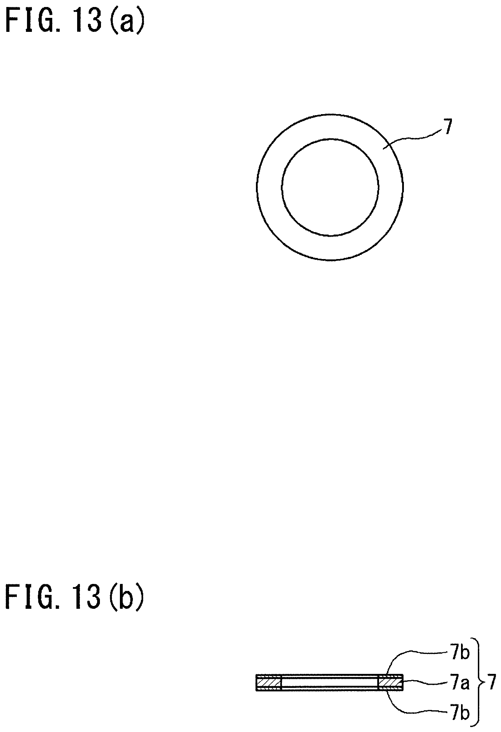

FIG. 13(a) is a plan view of a third double-sided tape 7 in the positioning tape 1 shown in FIG. 4, and FIG. 13(b) is a sectional view thereof. The third double-sided tape 7 has a planar shape substantially same as that of the marker sheet 4, and includes a base material 7a that is made of a synthetic resin such as polyethylene and is about 0.1 to 0.5 mm thick, and adhesive layers 7b that are provided on the both surfaces of the base material 7a and are made of a synthetic rubber adhesive agent.

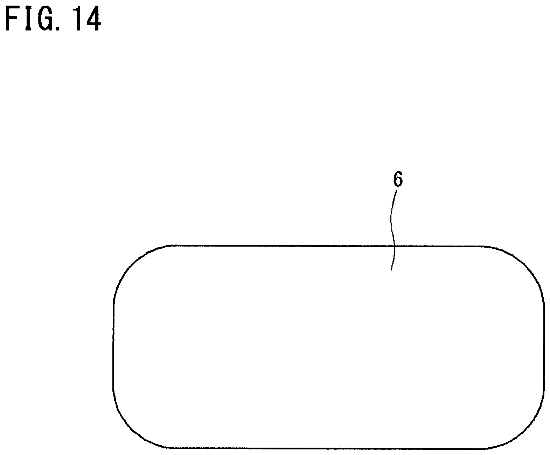

FIG. 14 is a plan view of the separable sheet 6 in the positioning tape 1 shown in FIG. 4. The separable sheet 6 is a substantially rectangular sheet having four chamfered corners, and can be made of a synthetic resin such as polyethylene terephthalate (PET) or paper material. The separable sheet 6 is not particularly limited in thickness in the present invention, but is ordinarily about 0.1 to 1.0 mm thick. The separable sheet 6 has a surface provided with silicone coating so as to be removed easily.

The longitudinal size (the size of the long side direction) of the separable sheet 6 is made larger by about 5 to 10 mm than the longitudinal size of the single-sided tape 5b. The separable sheet 6 and the single-sided tape 5b are positioned such that one of the longitudinal ends of the separable sheet 6 is extended from corresponding one of the longitudinal ends of the single-sided tape 5b. The portion of the separable sheet 6 extended from the single-sided tape 5b can be picked so as to easily remove the separable sheet 6 from the positioning tape 1.

The marker sheet 4 needs to reliably remain together with the supporter 5 when the separable sheet 6 is removed. The adhesive agents according to the present embodiment are selected so that adhesive force (adhesive strength) between the third double-sided tape 7 and the separable sheet 6 is smaller than adhesive force (adhesive strength) between the marker sheet 4 and the single-sided tape 5b. For the same reason, the adhesive agents are selected so that adhesive force (adhesive strength) between the third double-sided tape 7 and the separable sheet 6 is smaller than adhesive force (adhesive strength) between the marker sheet 4 and the third double-sided tape 7.

The remaining positioning tape 1 needs to be easily removed from the skin S whereas only the marker sheet 4 reliably remains on the skin S of the test subject after formation of micropores. The adhesive agents are selected so that adhesive force (adhesive strength) between the third double-sided tape 7 and the skin S is smaller than adhesive force (adhesive strength) between the marker sheet 4 and the single-sided tape 5b.

### [Tissue Fluid Collecting Method]

Described next is a tissue fluid collecting method with use of the positioning tape described above.

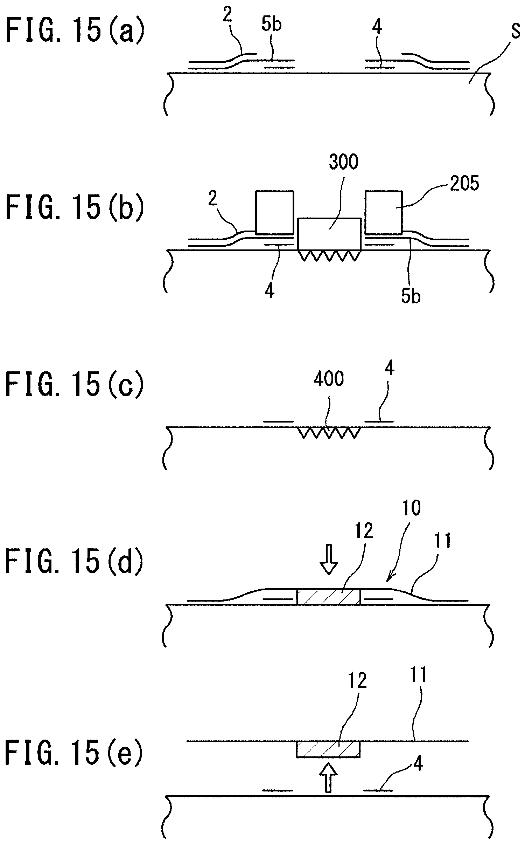

FIGS. 15 are explanatory views of the steps in the tissue fluid collecting method with use of the positioning tape described above.

The skin S of the test subject is initially cleaned by alcohol or the like to eliminate substances (sweat, dirt, and the like) disturbing acquisition of correct measurement results. The positioning tape 1 is then affixed to a predetermined position on the skin of the test subject (step (a)). FIGS. 15 show only the positioning portion 2, the single-sided tape 5b, and the marker sheet 4 in the positioning portion 1 for clearer depiction.

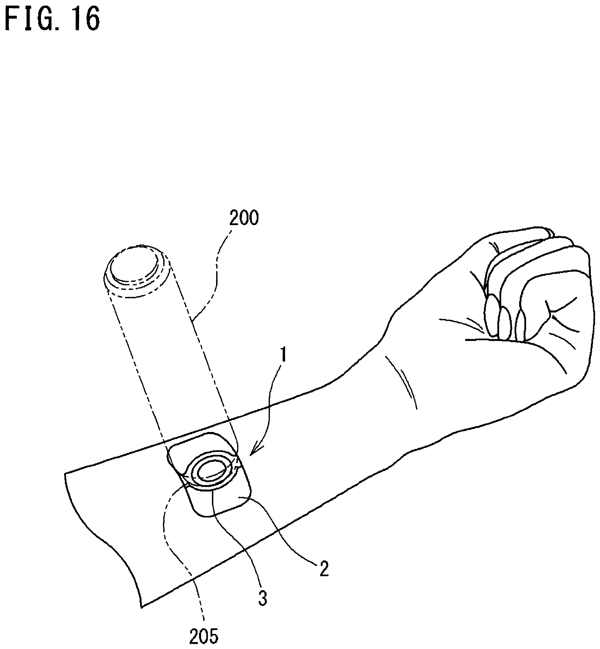

Micropores are then formed in the skin by the puncture tool 200 mounted with the microneedle chip 300 (step (b)). More specifically, as shown in FIG. 16, the puncture tool 200 is positioned by placing the contact portion 205 at the lower end of the puncture tool 200 so as to be matched with the positioning portion 2 in the positioning tape 1. In other words, the distal end of the contact portion 205 is placed in the circular arc steps 3 in the positioning portion 2 to position the puncture tool 200. The steps 3 regulate shift of the puncture tool 200 in the skin surface direction. The opening in the lower end surface of the contact portion 205 of the case 201 can be positioned accurately to be matched with the opening 4a in the marker sheet 4. When the release button 202 is pressed downward in this state, the fixing mechanism releases fixing of the array chuck 203 and bias force of the spring member 204 shifts the array chuck 203 toward the skin. The microneedle chip 300 mounted to the lower end of the array chuck 203 passes through the opening in the lower end surface of the contact portion 205 of the case 201 and comes into contact with a skin region of the test subject defined by the marker sheet 4. The micropores 400 are thus formed in the epidermis of the skin of the test subject.

The puncture tool 200 is then shifted away from the skin of the test subject, and the positioning portion 2 and the supporter 5 in the positioning tape 1 are removed from the skin S of the test subject (step (c)). As mentioned earlier, the positioning portion 2 is provided, in the vicinity of the marker sheet 4, with the plurality of notches 9 along the short sides of the positioning tape 1. The positioning portion 2 can be thus bent easily. Furthermore, the positioning portion 2 includes the two members. The supporter 5 can be thus removed easily from the skin S of the test subject.

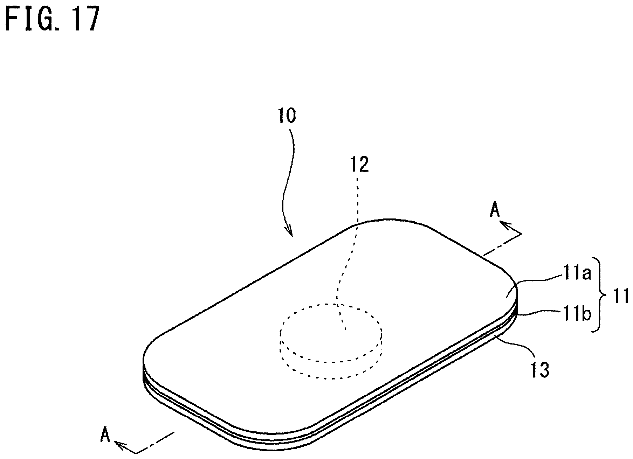

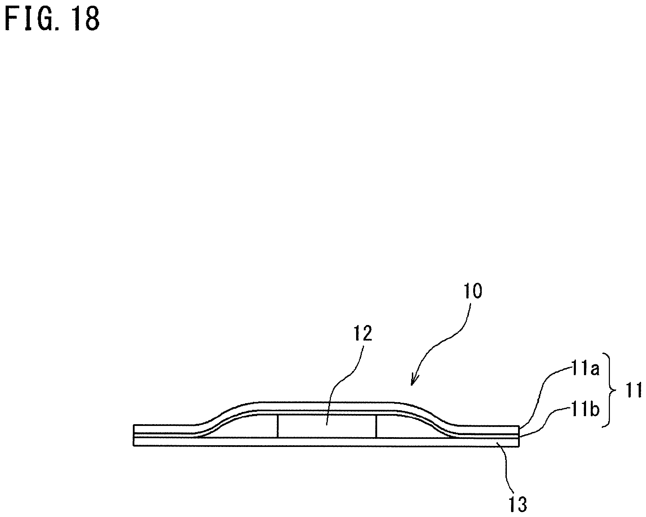

A tissue fluid collecting sheet 10 as shown in FIGS. 17 and 18 is subsequently affixed to the skin S of the test subject with reference to the marker sheet 4 as a mark so that a collector 12 is located in the opening 4a in the marker sheet 4 (step (d)). FIG. 17 is an explanatory perspective view of the tissue fluid collecting sheet 10. FIG. 18 is a sectional view taken along the line A-A of the tissue fluid collecting sheet 10 shown in FIG. 17.

The tissue fluid collecting sheet 10 includes a holder sheet 11 and the collector 12 held by the holder sheet 11.

The collector 12 is made of gel having a water holding property so as to hold tissue fluid extracted from the skin of the test subject, and contains pure water serving as an extraction medium. The gel is not particularly limited in the present invention and has only to collect tissue fluid. The gel is preferably made of at least one hydrophilic polymer selected from the group consisting of polyvinyl alcohol and polyvinyl pyrrolidone. The hydrophilic polymer configuring the gel can include only polyvinyl alcohol, only polyvinyl pyrrolidone, or a mixture thereof. The hydrophilic polymer more preferably includes only polyvinyl alcohol or the mixture of polyvinyl alcohol and polyvinyl pyrrolidone.

The gel can be obtained by crosslinking the hydrophilic polymer in an aqueous solution. The gel can be obtained by coating a base material with an aqueous solution of the hydrophilic polymer to form a coating film, and crosslinking the hydrophilic polymer contained in the coating film. Examples of a method of crosslinking a hydrophilic polymer include the chemical crosslinking method and the radiation crosslinking method. The radiation crosslinking method is preferably adopted because various chemical substances are unlikely to be mixed in gel as impurities.

The collector 12 according to the present embodiment has a short columnar shape. A surface of the collector 12 to be in contact with a skin is 10 mm in diameter and is thus smaller than the opening 4a in the marker sheet 4. The collector 12 can be thus located in the opening 4a without protruding from the opening 4a. This configuration can increase the ratio of the area of the collector 12 in contact with the micropore formation region, so that tissue fluid extracted through the micropores can be collected efficiently. The collector 12 is sized to be slightly larger than the surface provided with the microneedles of the microneedle chip. The micropore formation region can be thus fully utilized for collection of tissue fluid, and the test subject does not receive an excessive burden.

Similarly to the marker sheet 4, the preferred gel configuring the collector 12 is colored or has a colored intermediate layer so that the collector 12 is easily and accurately located in the opening 4a in the marker sheet 4. The intermediate layer is provided to improve an anchoring property of the gel to an adhesive surface, and is located directly on a surface of an adhesive agent layer. The preferred intermediate layer is a laminate of polyethylene terephthalate (PET) nonwoven fabric and a PET film, or a polyethylene film. The intermediate layer preferably has an area equal to the area of the gel provided to the skin.

The holder sheet 11 includes a sheet body 11a in an oval shape and an adhesive agent layer 11b provided on one of the surfaces of the sheet body 11a. The surface provided with the adhesive agent layer 11b serves as an adhesive surface. The collector 12 is located substantially at the center of a separable sheet 13 that also has an oval shape and serves also as a mount, and the holder sheet 11 is affixed to the separable sheet 13 so as to cover the collector 12. The collector 12 is held by part of the adhesive surface of the holder sheet 11. The holder sheet 11 has an area enough to cover the collector 12 in order to prevent the collector 12 from being dried upon collection of tissue fluid. In other words, the holder sheet 11 covers the collector 12, so that the skin and the holder sheet 11 can be kept airtight therebetween upon collection of tissue fluid and moisture contained in the collector 12 can be suppressed from evaporating upon collection of tissue fluid. The holder sheet 11 according to the present embodiment is made larger in area than the marker sheet 4 so as to fully cover the collector 12.

The sheet body 11a of the holder sheet 11 is colorless and transparent, or colored and transparent. The collector 12 held by the holder sheet 11 can be visually recognized easily from the front surface (opposite to the surface provided with the adhesive agent layer 11b) of the sheet body 11a. The sheet body 11a is preferably low in moisture permeability in order to prevent evaporation of tissue fluid or drying of the collector. Examples of the sheet body 11a include a polyethylene film, a polypropylene film, a polyester film, and a polyurethane film and the like. Preferred examples among them are the polyethylene film and the polyester film. The sheet body 11a is not particularly limited in thickness, but is about 0.025 to 0.5 mm thick.

The tissue fluid collecting sheet 10 is affixed to the skin by the adhesive surface of the holder sheet 11 so that the collector 12 is located in the micropore formation region in the opening 4a in the marker sheet 4. The collector 12 can be located easily in the micropore formation region with reference to the marker sheet 4 as a mark because the holder sheet holding the collector 12 is transparent.

The collector 12 located in the micropore formation region is left for a predetermined period of time such as at least 60 minutes, preferably at least 180 minutes, so that tissue fluid extracted through the micropores is collected at the collector 12. The holder sheet 11 holding the collector 12 is affixed to the skin S in the present embodiment. Even in a case where tissue fluid is collected for a long period of time such as 60 to 180 minutes, a user receives a less burden with no need to wear an assistive tool such as a belt around an arm.

After a predetermined period of time elapses from location of the collector 12 in the micropore formation region, the tissue fluid collecting sheet 10 is removed from the skin of the test subject (step (e)). The adhesive surface of the holder sheet 11 is affixed to the surface opposite to the adhesive surface of the marker sheet 4 by adhesive force smaller than adhesive force of the marker sheet 4 to the skin. The marker sheet 4 is not removed from the skin together with the holder sheet 11.

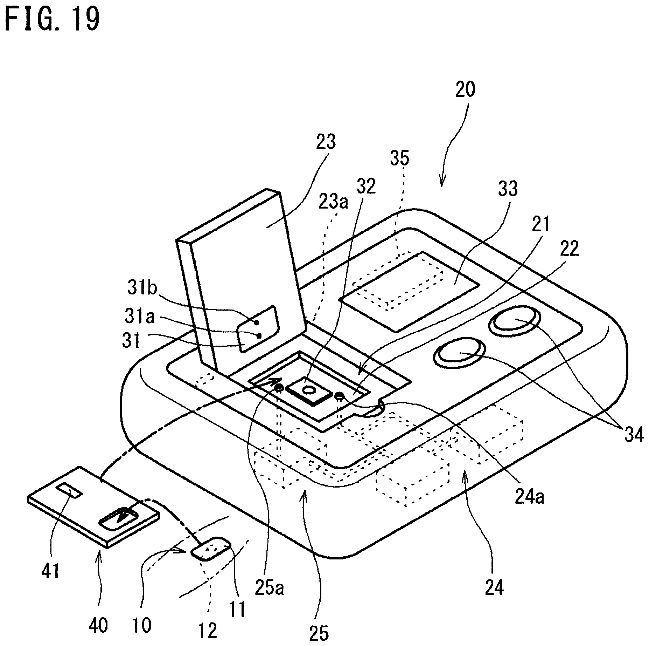

Tissue fluid collected at the collector 12 is subjected to componential analysis by an analyzer shown in FIG. 19, for example.

FIG. 19 is an explanatory perspective view of outer appearance of a biological component analyzer. A biological component analyzer 20 acquires a glucose concentration and a sodium ion concentration of the tissue fluid collected at the collector 12. The biological component analyzer 20 is used in the following manner. As indicated by dashed lines in FIG. 19, the tissue fluid collecting sheet 10 removed from the skin of the test subject is initially affixed to an analysis cartridge 40 that is placed in a cartridge location 22 of the biological component analyzer 20. The biological component analyzer 20 executes predetermined analysis on the analysis cartridge 40 placed in the cartridge location 22 and the tissue fluid collecting sheet 10 affixed to the analysis cartridge 40, to acquire a glucose concentration and a sodium ion concentration of the tissue fluid collected at the tissue fluid collecting sheet 10.

The biological component analyzer 20 includes a thick rectangular parallelepiped case that has a top plate provided with a concave portion 21. The concave portion 21 is provided with the cartridge location 22 that is further recessed from the concave portion 21. The concave portion 21 is connected to a movable top plate 23 having a thickness substantially same as the height of side walls of the concave portion 21. The movable top plate 23 is folded about a support shaft 23a so as to be accommodated in the concave portion 21 from the state shown in FIG. 19, or so as to be raised as shown in FIG. 19 from the state of being accommodated in the concave portion 21. The cartridge location 22 is large enough to accommodate the analysis cartridge 40 to be described later.

The movable top plate 23 is supported by the support shaft so as to be biased and accommodated in the concave portion 21. The analysis cartridge 40 placed in the cartridge location 22 is thus pressed from above by the movable top plate 23.

The biological component analyzer 20 includes therein a liquid conveyor 24 and a liquid discharger 25. The liquid conveyor 24 is a mechanism configured to convey liquid to the analysis cartridge 40 placed in the cartridge location 22, and injects liquid to the analysis cartridge 40 placed in the cartridge location 22 through a nipple 24a. The liquid discharger 25 is a mechanism configured to discharge liquid conveyed to the analysis cartridge 40 by the liquid conveyor 24, and discharges liquid having been injected to the analysis cartridge 40 through a nipple 25a.

The biological component analyzer 20 further includes a glucose detector 31, a sodium ion detector 32, a display 33, an operation unit 34, and a controller 35.

The glucose detector 31 is provided on the rear surface of the movable top plate 23, more specifically, on the surface facing the cartridge location 22 in the state where the movable top plate 23 is accommodated in the concave portion 21. The glucose detector 31 includes a light source 31a configured to emit light, and a light receiver 31b configured to receive reflected light of the light emitted from the light source 31a. The glucose detector 31 can irradiate, with light, the analysis cartridge 40 placed in the cartridge location 22, and can receive reflected light from the irradiated analysis cartridge 40. The analysis cartridge 40 contains a glucose reactant 41 that can chemically react with glucose in tissue fluid collected from a living body and be changed in color. The glucose detector 31 can detect a variation in absorbance by glucose in accordance with reflected light and quantitate the glucose from the reflected light thus obtained.

The sodium ion detector 32 is provided on the bottom surface of the cartridge location 22. The sodium ion detector 32 is provided, on the bottom surface of the cartridge location 22, with a rectangular plate member that is provided, substantially at the center, with a pair of sodium ion concentration measurement electrodes. The sodium ion concentration measurement electrodes comprise a sodium ion selective electrode that includes a sodium ion selecting film and is made of silver/silver chloride, and a silver/silver chloride electrode as a counter electrode.

The controller 35 is provided inside the biological component analyzer 20, and includes a CPU, a ROM, a RAM, and the like. The CPU reads out and executes a program stored in the ROM, to control operation of each unit. The RAM is utilized as a program development region for the program stored in the ROM and executed.

Operation of the biological component analyzer 20 thus configured is detailed next.

The tissue fluid collecting sheet 10 having collected tissue fluid is accommodated in an accommodating portion of the analysis cartridge 40. The analysis cartridge 40 is then placed in the cartridge location 22.

When execution of measurement is commanded, the liquid conveyor 24 injects liquid to the accommodating portion of the analysis cartridge 40 through the nipple 24a and the accommodating portion is filled with the liquid. Components in the tissue fluid disperse from the collector 12 to the liquid while the analysis cartridge is left for a predetermined period of time in this state. As described above, the marker sheet 4 is not removed from the skin together with the holder sheet 11 when the holder sheet 11 is removed from the skin. A stratum corneum component adhering to the marker sheet 4 is not dissolved in the liquid to affect measurement accuracy.

The liquid conveyor 24 conveys air to the accommodating portion after elapse of a predetermined period of time. When the air is conveyed, the liquid filling the accommodating portion is led to a flow path provided in the lower surface of the analysis cartridge 40 and further to the glucose reactant 41 through the flow path. The liquid led to the flow path comes into contact with the sodium ion detector 32. The liquid led to the glucose reactant 41 reacts with the glucose reactant 41 to change the glucose reactant in color.

The controller 35 acquires a voltage value between the sodium ion concentration measurement electrodes and acquires a sodium ion concentration based on the voltage value thus acquired and a calibration curve preliminarily stored in the controller 35.

The controller 35 acquires a glucose concentration based on a variation in amount between amount of light received by the light receiver 31b prior to color development of a color developing pigment and amount of light received by the light receiver 31b after color development of the color developing pigment.

### [Other Modification Examples]

The present invention is not limited to the embodiment described above, but can be modified in various manners.

For example, the positioning portion including the two sheets in the above embodiment can alternatively include a single sheet.

The marker portion is provided as the marker sheet separately from the supporter and only the marker sheet remains on the skin S after micropore formation in the above embodiment. When the single-sided tape serving as the supporter is provided, at the periphery of the opening, with a printed marker pattern, there is no need to provide the marker sheet. The printed portion of the single-sided tape serves as the marker portion in this case.

The positioning portion is provided separately from the supporter and the positioning portion is affixed to the single-sided tape serving as the supporter by the double-sided tape in the above embodiment. Alternatively, the single-sided tape and the positioning portion can be formed integrally of a synthetic resin or the like. When the single-sided tape is provided, at the periphery of the opening, with a printed marker pattern in this case, there is no need to provide the marker sheet.

The contact portion of the puncture tool has the circular outer shape and the steps in the positioning portion are shaped so as to correspond to the outer shape of the contact portion in the above embodiment. In a case where the contact portion of the puncture tool has a rectangular outer shape, the steps according to the present invention can be shaped so as to correspond to the rectangular shape.

Furthermore, the opening in the marker sheet has the circular shape in the above embodiment. The opening can alternatively have a different shape such as a rectangular shape so as to correspond to the shape of the microneedle chip of the puncture tool.

### REFERENCE SIGNS LIST

1: POSITIONING TAPE
2: POSITIONING PORTION
3: STEP
4: MARKER SHEET
4a: OPENING
5: SUPPORTER
5a: FIRST DOUBLE-SIDED TAPE
5b: SINGLE-SIDED TAPE
5c: THIN FILM PORTION
5d: SPACER
5e: SECOND DOUBLE-SIDED TAPE
6: SEPARABLE SHEET
7: THIRD DOUBLE-SIDED TAPE
9: NOTCH
10: TISSUE FLUID COLLECTING SHEET
12: COLLECTOR
20: BIOLOGICAL COMPONENT ANALYZER
200: PUNCTURE TOOL
205: CONTACT PORTION
300: MICRONEEDLE CHIP
301: MICRONEEDLE
400: MICROPORE
S: SKIN

## Claims

1. A positioning tape configured to indicate a region provided with a micropore when a skin is made in contact with a micropore forming device and is provided with the micropore by a microneedle of the micropore forming device, wherein the micropore forming device is contactable with the positioning tape (1), the positioning tape (1) comprising:
a supporter (5) having a positioning portion (2) including a step (3) having a shape at least partially corresponding to a shape of a contact portion (205) of the micropore forming device; and
a marker portion (4) having an opening corresponding to the region provided with the micropore;
**characterised in that**: when the contact portion (205) of the micropore forming device is made in contact with the positioning tape (1) for micropore formation, the step (3) at the positioning portion (2) is configured to regulate shift of the micropore forming device in a skin surface direction.

2. The positioning tape according to claim 1, wherein the marker portion (4) is provided as a marker sheet (4) separable from the supporter (5).

3. The positioning tape according to claim 2, wherein the marker sheet (4) has a circular shape.

4. The positioning tape according to any one of claims 1 to 3, wherein the positioning portion (2) is provided as a sheet that is separate from the supporter (5) and has a predetermined thickness.

5. The positioning tape according to claim 4, wherein the predetermined thickness is from 0.1 to 10.0 mm.

6. The positioning tape according to claim 4 or 5, wherein the positioning portion (2) includes a plurality of sheets.

7. The positioning tape according to any one of claims 1 to 6, wherein the step (3) at the positioning portion (2) has a circular shape or has at least partially circular arc shape.

8. The positioning tape according to any one of claims 1 to 7, wherein the positioning portion (2) is provided with a notch (9).

9. The positioning tape according to claim 8, wherein the notch (9) is provided along a short side of the positioning tape (1).

10. The positioning tape according to claim 8 or 9, wherein the positioning portion (2) includes a plurality of notches (9).

11. The positioning tape according to claim 2 or 3, wherein a notch (9) is provided in a vicinity of the marker sheet (4).

12. The positioning tape according to any one of claims 1 to 11, further comprising:
a separable sheet (6) provided on another surface of the supporter (5) to cover the marker portion (4); wherein
the separable sheet (6) is larger than the supporter (5) at one of ends in a long side direction.

13. The positioning tape according to any one of claims 1 to 12, wherein the supporter (5) has an opening corresponding to the region provided with the micropore.

14. The positioning tape according to any one of claims 1 to 12, wherein the supporter (5) is provided with a thin film portion (5c) in the region provided with the micropore.

15. The positioning tape according to claim 14, wherein the thin film portion (5c) is transparent.

## Patentansprüche

1. Positionierungsband, das dazu eingerichtet ist, einen Bereich anzudeuten, der mit einer Mikropore versehen wird, wenn Haut mit einer Mikroporenbildungsvorrichtung in Kontakt gebracht und durch eine Mikronadel der Mikroporenbildungsvorrichtung mit der Mikropore versehen wird, wobei die Mikroporenbildungsvorrichtung mit dem Positionierungsband (1) in Kontakt gebracht werden kann, wobei das Positionierungsband (1) umfasst:
einen Träger (5), der einen Positionierungsabschnitt (2) hat, der eine Stufe (3) enthält, die eine Form hat, die mindestens teilweise der Form eines Kontaktabschnitts (205) der Mikroporenbildungsvorrichtung entspricht; und
einen Markierungsabschnitt (4), der eine Öffnung hat, die dem mit der Mikropore versehenen Bereich entspricht;
**dadurch gekennzeichnet, dass**: wenn der Kontaktabschnitt (205) der Mikroporenbildungsvorrichtung zur Mikroporenbildung mit dem Positionierungsband (1) in Kontakt gebracht wird, die Stufe (3) an dem Positionierungsabschnitt (2) dazu eingerichtet ist, das Verschieben der Mikroporenbildungsvorrichtung in einer Hautoberflächenrichtung zu regulieren.

2. Positionierungsband nach Anspruch 1, wobei der Markierungsabschnitt (4) als ein von dem Träger (5) ablösbarer Markierungsbogen (4) vorgesehen ist.

3. Positionierungsband nach Anspruch 2, wobei der Markierungsbogen (4) eine Kreisform hat.

4. Positionierungsband nach einem der Ansprüche 1 bis 3, wobei der Positionierungsabschnitt (2) als Bogen vorgesehen ist, der von dem Träger (5) getrennt ist und eine vorgegebene Dicke hat.

5. Positionierungsband nach Anspruch 4, wobei die vorgegebene Dicke 0,1 bis 10,0 mm beträgt.

6. Positionierungsband nach Anspruch 4 oder 5, wobei der Positionierungsabschnitt (2) eine Vielzahl von Bogen enthält.

7. Positionierungsband nach einem der Ansprüche 1 bis 6, wobei die Stufe (3) an dem Positionierungsabschnitt (2) eine Kreisform hat oder eine mindestens teilweise Kreisbogenform hat.

8. Positionierungsband nach einem der Ansprüche 1 bis 7, wobei der Positionierungsabschnitt (2) mit einer Kerbe (9) versehen ist.

9. Positionierungsband nach Anspruch 8, wobei die Kerbe (9) entlang einer kurzen Seite des Positionierungsbandes (1) vorgesehen ist.

10. Positionierungsband nach Anspruch 8 oder 9, wobei der Positionierungsabschnitt (2) eine Vielzahl von Kerben (9) enthält.

11. Positionierungsband nach Anspruch 2 oder 3, wobei eine Kerbe (9) in der Nähe des Markierungsbogens (4) vorgesehen ist.

12. Positionierungsband nach einem der Ansprüche 1 bis 11, weiter umfassend:
einen ablösbaren Bogen (6), der an einer anderen Oberfläche des Trägers (5) vorgesehen ist, um den Markierungsabschnitt (4) abzudecken; wobei
der ablösbare Bogen (6) an einem der Enden in einer längsseitigen Richtung größer als der Träger (5) ist.

13. Positionierungsband nach einem der Ansprüche 1 bis 12, wobei der Träger (5) eine Öffnung hat, die dem mit der Mikropore versehenen Bereich entspricht.

14. Positionierungsband nach einem der Ansprüche 1 bis 12, wobei der Träger (5) in dem mit der Mikropore versehenen Bereich mit einem Dünnfilmabschnitt (5c) versehen ist.

15. Positionierungsband nach Anspruch 14, wobei der Dünnfilmabschnitt (5c) transparent ist.

## Revendications

1. Ruban de positionnement configuré pour indiquer une région pourvue d'un micropore lorsqu'une peau est mise en contact avec un dispositif de formation de micropore et est pourvue du micropore par une micro-aiguille du dispositif de formation de micropore, dans lequel le dispositif de formation de micropore peut être mis en contact avec le ruban de positionnement (1), le ruban de positionnement (1) comprenant :
un élément de support (5) ayant une partie de positionnement (2) incluant une marche (3) ayant une forme correspondant au moins partiellement à une forme d'une partie de contact (205) du dispositif de formation de micropore; et
une partie de marqueur (4) ayant une ouverture correspondant à la région pourvue du micropore ;
**caractérisé en ce que** : lorsque la partie de contact (205) du dispositif de formation de micropore est mise en contact avec le ruban de positionnement (1) pour une formation de micropore, la marche (3) au niveau de la partie de positionnement (2) est configurée pour réguler un déplacement du dispositif de formation de micropore dans une direction de surface de peau.

2. Ruban de positionnement selon la revendication 1, dans lequel la partie de marqueur (4) est prévue sous la forme d'une feuille de marquage (4) séparable de l'élément de support (5).

3. Ruban de positionnement selon la revendication 2, dans lequel la feuille de marquage (4) a une forme circulaire.

4. Ruban de positionnement selon l'une quelconque des revendications 1 à 3, dans lequel la partie de positionnement (2) est fournie sous la forme d'une feuille qui est séparée de l'élément de support (5) et présente une épaisseur prédéterminée.

5. Ruban de positionnement selon la revendication 4, dans lequel l'épaisseur prédéterminée est de 0,1 à 10,0 mm.

6. Ruban de positionnement selon la revendication 4 ou 5, dans lequel la partie de positionnement (2) inclut une pluralité de feuilles.

7. Ruban de positionnement selon l'une quelconque des revendications 1 à 6, dans lequel la marche (3) au niveau de la partie de positionnement (2) a une forme circulaire ou a une forme d'arc au moins partiellement circulaire.

8. Ruban de positionnement selon l'une quelconque des revendications 1 à 7, dans lequel la partie de positionnement (2) est pourvue d'une encoche (9).

9. Ruban de positionnement selon la revendication 8, dans lequel l'encoche (9) est prévue le long d'un côté court du ruban de positionnement (1).

10. Ruban de positionnement selon la revendication 8 ou 9, dans lequel la partie de positionnement (2) inclut une pluralité d'encoches (9).

11. Ruban de positionnement selon la revendication 2 ou 3, dans lequel une encoche (9) est prévue à proximité de la feuille de marquage (4).

12. Ruban de positionnement selon l'une quelconque des revendications 1 à 11, comprenant en outre :
une feuille séparable (6) fournie sur une autre surface de l'élément de support (5) pour couvrir la partie de marquage (4) ; dans lequel
la feuille séparable (6) est plus grande que l'élément de support (5) à l'une d'extrémités dans une direction de côté long.

13. Ruban de positionnement selon l'une quelconque des revendications 1 à 12, dans lequel l'élément de support (5) a une ouverture correspondant à la région pourvue du micropore.

14. Ruban de positionnement selon l'une quelconque des revendications 1 à 12, dans lequel l'élément de support (5) est pourvu d'une partie de film mince (5c) dans la région munie du micropore.

15. Ruban de positionnement selon la revendication 14, dans lequel la partie de film mince (5c) est transparente.
